(19) Europäisches Patentamt
European Patent Office
Office européen des brevets

(11) EP 2 467 151 B1

(12) FASCICULE DE BREVET EUROPEEN

(45) Date de publication et mention de la délivrance du brevet:
**14.09.2016 Bulletin 2016/37**

(21) Numéro de dépôt: **10723183.9**

(22) Date de dépôt: **01.04.2010**

(51) Int Cl.:
*A61K 35/644* (2015.01) *A23L 29/275* (2016.01)
*A61K 36/45* (2006.01) *A61P 31/04* (2006.01)

(86) Numéro de dépôt international:
**PCT/FR2010/050627**

(87) Numéro de publication internationale:
**WO 2011/020957 (24.02.2011 Gazette 2011/08)**

# (54) COMPOSITION ALIMENTAIRE ANTIBACTERIENNE

ANTIBAKTERIELLE LEBENSMITTELZUSAMMENSETZUNG

ANTIBACTERIAL FOOD COMPOSITION

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **21.08.2009 FR 0955738**

(43) Date de publication de la demande:
**27.06.2012 Bulletin 2012/26**

(73) Titulaire: **Nutrivercell**
**91000 Evry (FR)**

(72) Inventeur: **RENARD, Loïc**
**F-92500 Rueil Malmaison (FR)**

(74) Mandataire: **Lavoix**
**62, rue de Bonnel**
**69448 Lyon Cedex 03 (FR)**

(56) Documents cités:
**EP-A1- 1 902 721** **EP-A1- 1 913 951**
**WO-A2-02/062362** **US-A1- 2005 158 381**

- **Max Shrem: "Famille Mary: Offering pharmaceuticals made from honey" SLASHFOOD 18 juin 2008 (2008-06-18), pages 1-8, XP002574073 Extrait de l'Internet: URL:http://www.slashfood.com/2008/06/18/famille-mary-offering-pharmaceuticals-made-from-honey/ [extrait le 2010-03-19]**
- **BRUYERE ET AL: "Utilisation de la canneberge dans les infections urinaires recidivantes" MEDECINE ET MALADIES INFECTIEUSES, SOCIETE FRANCAISE D'EDITIONS MEDICALES, PARIS, FR, vol. 36, no. 7, 1 juillet 2006 (2006-07-01), pages 358-363, XP025087244 ISSN: 0399-077X [extrait le 2006-07-01]**
- **UZEL A ET AL: "Chemical compositions and antimicrobial activities of four different Anatolian propolis samples" MICROBIOLOGICAL RESEARCH, FISCHER, JENA, DE, vol. 160, no. 2, 25 avril 2005 (2005-04-25), pages 189-195, XP025323546 ISSN: 0944-5013 [extrait le 2005-04-25]**
- **POPOVA M ET AL: "Antibacterial activity of Turkish propolis and its qualitative and quantitative chemical composition" PHYTOMEDICINE, GUSTAV FISCHER VERLAG, STUTTGART, vol. 12, no. 3, 22 mars 2005 (2005-03-22), pages 221-228, XP025323698 ISSN: 0944-7113 [extrait le 2005-03-22]**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 467 151 B1

## Description

La présente invention concerne une composition alimentaire antibactérienne comprenant un extrait de *Propolis* et un extrait de *Vaccinium macrocarpon.* L'invention concerne plus particulièrement une composition alimentaire antibactérienne destinée au traitement des infections bactériennes aigues ou chroniques. L'invention concerne aussi l'association d'un extrait de *Propolis* ou d'une composition comprenant un extrait de *Propolis* et un extrait de *Vaccinium macrocarpon,* avec certaines classes d'antibiotiques.

Les infections urinaires sont d'une extrême fréquence. Elles viennent, après les infections respiratoires, au second rang des motifs de consultation et de prescription d'antibiotiques. Elles sont facilement récidivantes notamment chez la femme et les porteurs de vessie neurologique. L'agent microbien responsable à plus de 90% de ces infections urinaires est *Escherichia coli,* bactérie commensale du tube digestif qui peut devenir pathogène par acquisition d'îlots de pathogénicité.

Leur prise en charge par la thérapeutique classique, et en particulier par le recours aux antibiotiques, s'est généralisée mais s'avère cependant de moins en moins satisfaisante, étant donné la résistance croissante des bactéries à ces agents antibiotiques.

Pour résoudre ce problème, une des solutions est de diminuer la population bactérienne par des mécanismes très différents de ceux des antibiotiques et qui agissent donc de façon indépendante de la résistance et de la virulence des bactéries.

Le *Vaccinium macrocarpon* est une plante fruitière correspondant à la cranberry ou canneberge. L'extrait de cranberry *Vaccinium macrocarpon* est connu pour ses propriétés antiadhérentielles bactériennes qui ont été mises à profit dans le traitement de nombreuses infections urinaires pour combattre le développement des bactéries. La consommation de jus de cranberry *Vaccinium macrocarpon* préviendrait les infections urinaires (JP Lavigne, Clin. Microbiol. Infect. 2007, p1-5). La société Tournay commercialise des extraits sous la dénomination Exocyan®, caractérisés par leur teneur en proanthocyanidines (PAC), standardisée à des valeurs de 10 à 50% selon la méthode de mesure Vanilline.

La demande de brevet EP 1 902 721 décrit l'association d'un extrait de *Vaccinium macrocarpon* et d'un agent antibactérien urinaire contre les bactéries à Gram+ et à Gram- dans une composition destinée à lutter contre les infections urinaires et à être sélective pour ne pas faire disparaître la flore bactérienne normalement présente dans l'urètre.

Les propriétés à la fois bactériostatiques et bactéricides de la *Propolis* sur de nombreuses souches microbiennes, et en particulier sur *Escherichia coli* sont également connues.

Trois compléments nutritionnels destinés au traitement des infections urinaires sont commercialisés sous les noms Urimel par la société Famille Mary, "Biophyto.com Infection Urinaire gènes urinaires fréquentes récidivantes" par la société Biophyto.com et Uriprop par la société Laboratoire Lax et comprennent un extrait de canneberge associé à un extrait de *Propolis,* sans plus de précision.

Le praticien manque aujourd'hui d'informations, pourtant essentielles, d'une part, sur la provenance des extraits et leur traitement ou transformation, qui conditionnent pour une grande part l'activité du complément alimentaire, et d'autre part, sur les teneurs dans les principes actifs essentiels de chacun de ces extraits. Le praticien enfin manque aussi d'informations sur les conséquences de l'association de ces extraits. Ce manque d'informations de fait limite l'usage qu'il est possible de faire de ces extraits, limitant du coup cet usage à des produits peu caractérisés ne répondant pas à la règlementation des compléments alimentaires en vigueur.

L'invention a au contraire pour objectif de proposer des compléments alimentaires caractérisés et reproductibles, permettant un usage en pratique quotidienne, et notamment donnant au praticien, au médecin, la possibilité de prescrire un traitement du type complément alimentaire, seul ou en association avec un traitement thérapeutique classique afin d'en accroître l'efficacité ou d'en retrouver l'efficacité originelle.

Ainsi un premier objectif de l'invention consiste à proposer une composition qui s'affranchisse des inconvénients connus de l'état de la technique mentionnés ci-dessus.

Un autre objectif de l'invention est de proposer une composition antibactérienne dont l'efficacité sur le traitement et/ou la prévention des infections bactériennes aigues ou chroniques, et en particulier sur le traitement des infections urinaires, est améliorée.

Un autre objectif de l'invention est de proposer une composition qui permet de palier un éventuel déficit en zinc et en fer qui peut se présenter respectivement chez l'homme et la femme.

Un autre objectif de l'invention est de proposer une composition qui permet d'améliorer la biodisponibilité de la vitamine C à des doses nutritionnelles en évitant ainsi une surconsommation de vitamine C.

La présente invention a pour objet une composition alimentaire antibactérienne comprenant un extrait de cranberry *Vaccinium macrocarpon* et un extrait de *Propolis* dans laquelle :

- l'extrait *de Propolis* comprend de l'acide caféique, de l'acide férulique, de la galangine et de la pinocembrine,
- l'extrait de cranberry *Vaccinium macrocarpon* contient une proportion en poids de proanthocyanidines (PAC) supérieure ou égale à 10%, de préférence supérieure à 10%, avantageusement comprise entre 20 et 50%, ladite

proportion étant mesurée par la méthode de dosage par la Vanilline,

la composition étant caractérisée par un ratio en poids extrait de *Propolis :* extrait de cranberry *Vaccinium macrocarpon* compris entre 1 :2 et 2 :1.

La *Propolis* désigne toute une série de substances résineuses, gommeuses et balsamiques, de consistance visqueuse, recueillies sur certaines parties, notamment les bourgeons et les écorces, de végétaux par les abeilles qui les rapportent à la ruche et les modifient en partie avec l'ajout de leurs propres sécrétions salivaires et de cire. Ces végétaux sont principalement des arbres tels que pins, sapins, épicéas, peupliers, aulnes, saules, marronniers d'inde, bouleaux, pruniers, frênes, chênes ou encore ormes.

Par extrait de *Propolis,* on entend une forme de *Propolis* pouvant être mise en oeuvre. Elle peut donc être non transformée ou traitée, ou bien transformée en présence notamment d'un excipient approprié, par exemple caroube, amidon ou dérivé d'amidon, e.g. maltodextrine. La *Propolis* peut notamment se présenter sous la forme d'une poudre. Pour cela, la *Propolis* peut être mélangée à une solution hydro alcoolique à laquelle est ajouté un excipient, tel que la maltodextrine ou la poudre de caroube, en tant que support. Le mélange ainsi obtenu est évaporé puis séché. Un extrait de *Propolis* comprenant 18% de *Propolis* et 82% de poudre de caroube est en outre commercialisé sous le nom Propolis PPM 18 par la société LUSTREL. Un autre exemple d'extrait de *Propolis* comprenant 60% de *Propolis* et 40% de caroube est commercialisé par la société CLAUDINE VALLEE.

Dans un mode de réalisation préféré, la teneur en poids d'extrait de *Propolis* dans la composition selon l'invention est comprise entre 10 et 80%, de préférence entre 30 et 70%.

L'extrait de cranberry *Vaccinium macrocarpon* est de préférence obtenu par un procédé de concentration du totum des fractions polyphénoliques du jus de cranberry *Vaccinium macrocarpon.*

Le jus de cranberry *Vaccinium macrocarpon* est tiré d'un fruit cultivé essentiellement dans le Nord des Etats-Unis, ainsi qu'au Canada. Ce jus de couleur rouge sombre et de saveur astringente est riche en polyphénols et notamment en proanthocyanidines sous forme libre glycosylée (galactoside, rhamnoside, glucoside) ou estérifiée (gallate) comme par exemple la prodelphinidine, la propetunidine, la promalvidine ou la procyanidine. Ces composés existent en général sous forme polymérisée (dimères à hexamères) et possèdent un enchaînement de type A ou de type B selon les deux schémas suivants, correspondant aux structures d'un dimère de type B et d'un dimère de type A :

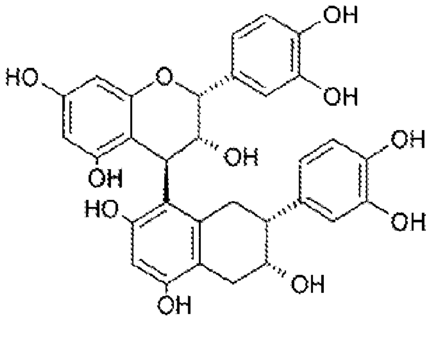

a) dimère de type B

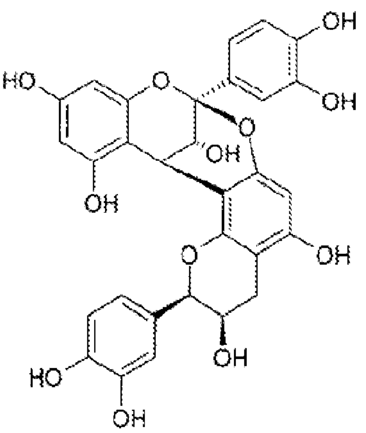

b) dimère de type A

Le procédé de concentration et d'isolement des fractions polyphénoliques du jus de cranberry *Vaccinium macrocarpon* consiste notamment à éliminer les substances inertes telles que fibres, sucres et acides de fruit du jus de cranberry *Vaccinium macrocarpon* par chromatographie sur résine échangeuse d'ions. Ce procédé fixe les polyphénols et laisse ainsi non adsorbées toutes les substances inertes présentes dans le jus.

La résine échangeuse d'ions consiste de préférence en une résine polymérique non ionique, telle que la résine commercialisée sous la dénomination Amberlite® XAD 16 HP par la société ROHM & HAAS. Cette dernière est constituée d'un copolymère styrène/divinylbenzène réticulé aliphatique et se présente sous forme de billes blanches translucides retenant de l'humidité (62-70 %). Elle possède une surface spécifique de 800 $m^2/g$ et une porosité de ≥ 0,55 ml/ml. Ce type de résine est neutre et convient pour n'importe quelle zone de pH. La taille des pores en volume est d'environ 1,082 ml/g et la taille moyenne des pores est d'environ 100 Å.

D'autres résines telles que les résines Amberlite XAD 4, Amberlite XAD 16 ou Amberlite XAD 2 commercialisées par la société ROHM & HAAS peuvent également être utilisées.

On procède ensuite à une élution des polyphénols. L'éthanol permet d'éluer progressivement les polyphénols

sans les altérer et la récupération de l'éluat est poursuivie jusqu'à disparition de la coloration violette dans l'éluat. On effectue de préférence un dosage des polyphénols dans l'éluat pour s'assurer que la totalité des polyphénols a bien été éluée.

Selon une modalité particulière du procédé, le rinçage de la colonne s'effectue avec de l'eau pour éliminer les composés non retenus par la résine. Le rinçage de la colonne est stoppé quand le taux de matière sèche dans l'éluat est inférieur à 1 %.

L'éluat peut subir une lyophilisation ou une atomisation permettant ainsi d'obtenir un extrait de cranberry *Vaccinium macrocarpon* sous forme d'une poudre.

La proportion en proanthocyanidines (PAC) de l'extrait de cranberry *Vaccinium macrocarpon* est mesurée par la méthode de dosage par la Vanilline.

Le principe de ce dosage est basé sur la fixation de l'aldéhyde de la vanilline sur le carbone 6 du cycle A de la catéchine pour former un complexe chromophore rouge qui absorbe à 500 nm. La vanilline réagit avec les monomères catéchiques et les unités terminales des PAC alors qu'elle ne réagit pas avec les unités intermédiaires des PAC étant donné que leur carbone 6, site de fixation de la vanilline, est pris dans la liaison monomère-monomère (C4-C6).

On pèse x mg d'extrait de cranberry *Vaccinium macrocarpon* (environ 20 mg pour une poudre ou 50 mg pour un liquide) que l'on dilue dans 100 ml d'eau à pH 2 (le pH étant ajusté avec de l'acide chlorhydrique (HCl) à 37% en utilisant un pHmètre). 1 ml de solution d'extrait de cranberry *Vaccinium macrocarpon* est introduit dans un tube à essai de 15 ml avec bouchon rodé. A ce volume on ajoute successivement entre 3 et 12 ml de réactif vanillique comprenant 4 % de vanilline dans du méthanol et entre 3 et 12 ml d'acide chlorhydrique concentré à 37% (HCl) (BURNS 1971). La solution obtenue est agitée pendant 20 min à 30 °C dans un bain-marie. Après 20 min, on mesure l'absorbance A à 500 nm de la solution, en utilisant comme liquide de compensation une solution identique à la solution dont l'absorbance A est mesurée et dans laquelle le réactif vanillique est remplacé par du méthanol pur. On prépare une gamme standard en utilisant la catéchine. Pour cela, on prépare 3 solutions de catéchine en pesant respectivement 5 mg, 10 mg et 15 mg que l'on introduit dans une fiole jaugée de 100 ml de méthanol, chacune de ces 3 solutions étant stable pendant une journée. Le dosage du standard est effectué sur 1 ml de chacune des solutions de la gamme standard en procédant de la même façon que pour l'échantillon. La teneur en proanthocyanidines totaux exprimés en équivalent catéchine pour 100 g de poudre d'extrait de cranberry *Vaccinium macrocarpon* est calculée à l'aide de la formule suivante :

$$[\text{proanthocyanidines}]\% = \frac{100 \times [\text{eq.catechine}]}{\dfrac{x}{0{,}1}}$$

Avec :

- [proanthocyanidines] teneur en proanthocyanidines,
- [eq.catechine] obtenue par la détermination de l'équation de la courbe d'absorption à 500 nm de la solution comprenant l'extrait de cranberry *Vaccinium macrocarpon* par régression linéaire,
- x : masse d'extrait de cranberry *Vaccinium macrocarpon*

Un extrait de cranberry *Vaccinium macrocarpon* comprenant 20% en PAC est en outre commercialisé sous le nom Exocyan CRAN 20S par la société Tournay Biotechnologies.

Dans un mode de réalisation particulier, la teneur en poids d'extrait de cranberry *Vaccinium macrocarpon* dans la composition selon l'invention est comprise entre 10 et 80%, de préférence entre 25 et 70%.

Dans un autre mode de réalisation, la composition selon l'invention comprend également un composé choisi dans le groupe constitué par les actifs pharmaceutiques, les ingrédients alimentaires et les nutriments.

Dans un mode de réalisation préféré, l'ingrédient alimentaire est choisi dans le groupe constitué par le zinc et ses dérivés.

Dans un mode de réalisation préféré, les dérivés du zinc sont choisis parmi les sels de zinc, tels que le citrate de zinc, l'oxyde de zinc, le sulfate de zinc, l'acétate de zinc ou le gluconate de zinc.

Dans un mode de réalisation préféré, la teneur en poids de zinc ou d'un de ses dérivés dans la composition selon l'invention est comprise entre 0,01 et 2%, de préférence entre 0,1 et 1,5%.

Dans un autre mode de réalisation préféré, l'ingrédient alimentaire est choisi dans le groupe constitué par le fer et ses dérivés.

Dans un mode de réalisation préféré, les dérivés du fer sont choisis parmi les sels de fer, tels que le sulfate de fer, le lactate de fer, le gluconate de fer, le chlorure de fer ou le fumarate de fer.

Dans un mode de réalisation préféré, la teneur en poids de fer ou d'un de ses dérivés dans la composition

selon l'invention est comprise entre 0,05 et 3%, de préférence entre 0,5 et 2,5%.

Dans un autre mode de réalisation, l'ingrédient alimentaire est choisi dans le groupe constitué par l'acide ascorbique communément dénommé vitamine C et ses dérivés.

Dans un mode de réalisation préféré, les dérivés de vitamine C sont choisis parmi les sels d'acide ascorbique, tels que l'ascorbate de calcium, l'ascorbate de magnésium, l'ascorbate de potassium ou l'ascorbate de sodium.

Dans un mode de réalisation préféré, le sel d'acide ascorbique est l'ascorbate de calcium.

Dans un mode de réalisation préféré, la teneur en poids de vitamine C ou d'un de ses dérivés dans la composition selon l'invention est comprise entre 1 et 10%, de préférence entre 2 et 8%.

Les teneurs en poids dans la composition selon l'invention de zinc et ses dérivés, de fer et ses dérivés ainsi que de vitamine C et ses dérivés respectent les apports journaliers recommandés pour chacun d'eux. La composition selon l'invention caractérisée par ces teneurs en poids peut représenter ainsi entre 50 et 85% des apports journaliers recommandés en zinc, fer ou vitamine C.

La composition selon l'invention peut comprendre en outre un excipient ou un vecteur inerte, non toxique. On pourra citer comme excipient inerte les sucres comme le lactose ou le fructose, la cellulose, le carbonate de calcium, le phosphate tricalcique, le phosphate de magnésium, le stéarate de calcium, le stéarate de magnésium, le talc ou la silice colloïdale (Aerosil (R) 100 ou Aerosil (R) 200 commercialisé par la société Degussa). Comme vecteurs, on pourra citer encore des composés qui favorisent l'excrétion urinaire comme par exemple l'extrait de Fumeterre ou l'extrait d'Orthosiphon.

La composition selon l'invention peut également comprendre un composé choisi dans le groupe constitué par les polyols comme la glycérine ou le sorbitol, les colorants, les édulcorants comme le sucralose ou les actifs aromatiques comme les arômes de fruit.

La composition selon l'invention se présente sous forme solide ou liquide.

Dans un mode de réalisation particulier, la composition selon l'invention se présente sous forme de poudre, de gélules, de capsules, de gommes à mâcher, de granulés, de granules, de cachets, de pilules, de pastilles, de comprimés ou de tablettes. La mise en oeuvre galénique est effectuée dans des conditions de températures et de pression qui respectent l'intégrité des ingrédients mis en oeuvre et la bioactivité des ingrédients actifs.

Les compositions selon l'invention sont destinées en premier lieu à développer un effet physiologique favorable chez des personnes souffrant ou sujettes à des infections bactériennes, ainsi que celles présentant des bactériuries asymptomatiques ou présentant un risque de bactériuries asymptomatiques, notamment les femmes enceintes, les femmes âgées, les homme souffrant d'adénome de la prostate, les personnes handicapées ou les personnes après un acte diagnostique médical ou une intervention chirurgicale. Cet effet physiologique a un effet favorable permettant de prévenir et/ou limiter ces infections ou ces bactériuries. Les effets physiologiques les plus marquants sont l'inhibition de la croissance bactérienne, un effet bactéricide ou bactériostatique, un effet de diminution de l'adhérence des bactéries, en particulier des entérobactéries.

Suivant une modalité de l'invention, la composition a un effet dit "bacterioflush". On entend par effet dit "bactérioflush" ou effet dit "chasse d'eau" tout effet par lequel les bactéries sont éliminées de l'organisme par les voies naturelles.

Les compositions selon l'invention sont particulièrement utiles chez des personnes souffrant d'infections vésicales ou urinaires, par exemple la femme à risque ou antériorité de cystites, en particulier enceinte, l'homme souffrant d'adénome bénin de la prostate, en particulier de plus de 50 ans, ainsi que les personnes souffrant d'infections buccales, gastriques et/ou respiratoires ou encore les personnes souffrant de stases urinaires, en particulier les personnes âgées ou handicapées.

L'effet antiadhérentiel est particulièrement intéressant pour intervenir tôt lors d'une infection et diminuer la charge bactérienne par l'effet d'élimination des bactéries, ou encore pour diminuer le risque d'apparition d'une infection chez le sujet sensible. La composition peut aussi avoir un effet de diminution de la virulence des bactéries à pili P, par diminution de l'expression de ces pili.

Dans un mode réalisation particulier, la composition selon l'invention permet de diminuer l'expression de gènes de virulence des souches bactériennes uropathogènes et donc de diminuer la virulence de ces souches. Par exemple, la composition selon l'invention permet de diminuer l'expression du gène *papG3.*

Un second objet de la présente demande concerne donc l'utilisation d'une composition selon l'invention pour diminuer l'expression des gènes de virulence de bactéries à pili P, notamment des bactéries uropathogènes, et plus particulièrement *Escherichia Coli.*

La composition selon l'invention est notamment caractérisée par un effet antiadhérentiel qui se maintient dans le temps, jusqu'à 24h. Une des explications avancées pour expliquer cet effet est la potentialisation de l'effet antiadhérentiel des PAC présents dans l'extrait de cranberry *Vaccinium macrocarpon* par l'extrait de l'extrait de *Propolis.*

La divulgation concerne aussi une composition comprenant un extrait de *Propolis* et un extrait de cranberry *Vaccinium macrocarpon,* pour une administration simultanée, séparée ou étalée dans le temps, pour le traitement et/ou la prévention des infections bactériennes aigues ou chroniques ou des bactériuries asymptomatiques, et notamment

des infections vésicales ou urinaires

La divulgation concerne aussi une utilisation dans une méthode de traitement comprenant l'administration séparée, simultanée ou étalée dans le temps, par voie orale chez un mammifère, notamment un humain, d'une composition comprenant un extrait de *Propolis* et d'une composition comprenant un extrait de cranberry *Vaccinium macrocarpon,* pour le traitement et/ou la prévention des infections bactériennes aigues ou chroniques ou des bactériuries asymptomatiques, et notamment des infections vésicales ou urinaires.

La divulgation prévoit en effet aussi l'utilisation d'une composition comprenant un extrait de *Propolis* pour précéder, complémenter et/ou suivre l'utilisation d'une composition comprenant un extrait de cranberry *Vaccinium macrocarpon.*

Selon la dose administrée, les compositions peuvent aussi avoir un effet pharmacologique.

Un troisième objet de la présente demande concerne un complément alimentaire ou un médicament comprenant une composition selon l'invention pour l'utilisation dans le traitement et/ou la prévention des infections bactériennes aigues ou chroniques ou des bactériuries asymptomatiques.

Dans un mode de réalisation préféré, le complément alimentaire ou le médicament selon l'invention est destiné au traitement et/ou à la prévention des infections vésicales ou des infections urinaires.

Dans un autre mode de réalisation préféré, le complément alimentaire ou le médicament selon l'invention est destiné au traitement et/ou à la prévention des infections buccales, gastriques ou respiratoires.

Dans un autre mode de réalisation préféré, le complément alimentaire ou le médicament selon l'invention est destiné au traitement et/ou à la prévention des infections vésicales ou urinaires associées à un éventuel déficit en zinc chez l'homme, en particulier chez l'homme de plus de cinquante ans, notamment souffrant d'adénome bénin de la prostate.

Dans un autre mode de réalisation préféré, le complément alimentaire ou le médicament selon l'invention est destiné au traitement et/ou à la prévention des infections vésicales ou urinaires associées à un éventuel déficit en fer chez la femme, en particulier la femme à risque ou antériorité de cystites et notamment chez la femme enceinte.

Dans un autre mode de réalisation préféré, le complément alimentaire ou le médicament selon l'invention est destiné au traitement et/ou à la prévention des stases urinaires chez les personnes âgées ou handicapées.

Dans un autre mode de réalisation préféré, le complément alimentaire ou le médicament selon l'invention est destiné au traitement et/ou à la prévention des infections bactériennes aigues ou chroniques et au renforcement du système immunitaire.

Un quatrième objet de la présente divulgation concerne les méthodes de traitement comprenant l'administration par voie orale chez un mammifère, notamment un humain, d'une composition selon l'invention pour le traitement et/ou la prévention des infections bactériennes aigues ou chroniques ou des bactériuries asymptomatiques.

La méthode de traitement comprend l'administration par voie orale d'une composition selon l'invention pour le traitement et/ou la prévention des infections vésicales ou des infections urinaires.

La méthode de traitement comprend l'administration par voie orale d'une composition selon l'invention pour le traitement et/ou la prévention des infections buccales, gastriques ou respiratoires.

La méthode de traitement comprend l'administration par voie orale d'une composition selon l'invention pour le traitement et/ou la prévention des infections urinaires ou vésicales associées à un éventuel déficit en zinc chez l'homme en particulier chez l'homme de plus de cinquante ans, souffrant notamment d'adénome bénin de la prostate.

La méthode de traitement comprend l'administration par voie orale d'une composition selon l'invention pour le traitement et/ou la prévention des infections urinaires ou vésicales associées à un éventuel déficit en fer chez la femme, en particulier chez la femme à risque ou antériorité de cystites, notamment chez la femme enceinte.

La méthode de traitement comprend l'administration par voie orale d'une composition selon l'invention pour le traitement et/ou la prévention des stases urinaires chez les personnes âgées ou handicapées.

La méthode de traitement comprend l'administration par voie orale d'une composition selon l'invention pour le traitement et/ou la prévention des infections bactériennes aigues ou chroniques et le renforcement du système immunitaire.

Ce traitement peut être associé à un traitement thérapeutique antibactérien, notamment à l'administration d'un antibiotique, tels que ofloxacine (fluoroquinolone), ceftriaxone et cefixime (céphalosporine), les β-lactamines et la nitrofuradantine. La divulgation a donc pour objet l'utilisation de la composition selon l'invention pour précéder, complémenter et/ou suivre un traitement thérapeutique antibactérien. Cela signifie que le praticien prescrit au patient qui en a besoin de consommer le complément alimentaire ou le médicament selon l'invention en cure avant, pendant et/ou après la conduite d'un traitement par antibactérien, par exemple antibiotique.

L'invention a aussi pour objet un traitement dit flash, consistant à participer à l'élimination des germes résistant à l'ATB (antibiothérapie) mais aussi à diminuer de façon significative la population bactérienne par un effet Flush (effet chasse d'eau). Les bactéries sont évacuées par les voies naturelles et l'expression phylogénétique du système d'adhésion et plus particulièrement l'expression de virulence des Pili de type P est diminuée.

On administre une dose efficace journalière de la composition selon l'invention. Par dose efficace journalière,

on entend une dose de la composition selon l'invention consommée sur une période de 24 heures.

Par dose journalière efficace, on entend notamment une quantité de composition selon l'invention qui comprend entre 300 et 1200 mg d'extrait de cranberry *Vaccinium macrocarpon* et entre 400 et 2000 mg d'extrait de *Propolis.*

L'invention a encore pour objet une composition comprenant un extrait de *Propolis* et un extrait de cranberry *Vaccinium macrocarpon* et un principe actif pharmaceutique, par exemple antibactérien, antibiotique, antioxydant comme la vitamine C et ses dérivés ou un ingrédient alimentaire comme le zinc, le fer et leurs dérivés ou un nutriment, pour une administration simultanée, séparée ou étalée dans le temps.

Un autre objet de la présente demande concerne un kit comprenant

- une première composition comprenant un extrait de *Propolis* et un extrait de cranberry *Vaccinium macrocarpon,* selon l'invention
- une seconde composition comprenant un principe actif pharmaceutique par exemple antibactérien, antibiotique, antioxydant comme la vitamine C et ses dérivés ou un ingrédient alimentaire comme le zinc, le fer et leurs dérivés ou un nutriment.

Les différentes caractéristiques présentées pour l'extrait de *Propolis* et l'extrait de cranberry *Vaccinium macrocarpon* s'appliquent également au kit selon l'invention cité précédemment.

Les différentes caractéristiques présentées pour le zinc et ses dérivés, le fer et ses dérivés et la vitamine C et ses dérivés s'appliquent également au kit selon l'invention cité précédemment.

Dans le cadre de l'association de la composition selon l'invention avec différents antibiotiques, il a été découvert un effet de synergie de la *Propolis* en l'absence de cranberry *Vaccinium macrocarpon* avec certaines classes d'antibiotiques, et notamment ceux utilisés en pathologie urinaire. Cette synergie permet d'améliorer l'activité antibactérienne de l'antibiotique, sans en avoir à augmenter la quantité administrée.

Cette synergie permet donc de limiter voir de diminuer, dans le cas d'un traitement thérapeutique antibactérien, et notamment dans le cas d'un traitement d'infections urinaires, la dose d'antibiotiques administrée ainsi que la fréquence d'administration chez le patient. Elle permet de réduire les risques de développement de résistance à ces antibiotiques chez ce patient.

Au sens de l'invention, « simultanée » signifie que les deux actifs sont administrés par la même voie en même temps (par exemple ils sont mélangés), « séparée » signifie qu'ils sont administrés par des voies différentes ou en des lieux différents, et « étalée dans le temps » signifie qu'ils sont administrés séparément à des instants différents.

Les différentes caractéristiques présentées pour l'extrait de *Propolis* s'appliquent également à l'invention citée précédemment.

Dans la présente invention, chaque fois que l'on parle d'antibiotique, il faut entendre tout agent antibiotique pouvant être choisi parmi l'ensemble des classes d'antibiotiques connues, et notamment parmi les classes d'antibiotiques utilisées en pathologie urinaire, et qui pour lesquelles l'association à la *Propolis* et à la cranberry *Vaccinium macrocarpon* se révèle utile. On peut citer avantageusement les céphalosporines et plus particulièrement les céphalosporines de troisième génération comme le ceftriaxone ou le cefixime et les fluoroquinolones comme l'ofloxacine. On peut aussi citer les $\beta$-lactamines et la nitrofuradantine.

Les différentes caractéristiques présentées concernant les formes que peut prendre la composition ainsi que les composés additionnels s'appliquent également à la divulgation.

Un autre objet de la divulgation est une telle composition pour le traitement et/ou la prévention des infections bactériennes aigues ou chroniques ou des bactériuries asymptomatiques, et notamment des infections vésicales ou urinaires.

Un autre objet de la divulgation est une méthode de traitement antibactérien comprenant l'administration chez un mammifère, notamment un humain, d'une telle composition pour le traitement et/ou la prévention des infections bactériennes aigues ou chroniques ou des bactériuries asymptomatiques, et notamment des infections vésicales ou urinaires.

la divulgation a donc pour objet l'utilisation d'une composition comprenant un extrait de *Propolis* pour précéder, complémenter et/ou suivre un traitement antibiotique. Cela signifie que le praticien prescrit au patient qui en a besoin de consommer la composition comprenant l'extrait de *Propolis* en cure avant, pendant et/ou après la conduite d'un traitement antibiotique.

Les différentes caractéristiques présentées pour l'extrait de *Propolis* et l'agent antibiotique ainsi que celles concernant les formes que peut prendre la composition s'appliquent également à la divulgation citée précédemment.

Les présentes inventions et leurs différents modes de réalisation seront mieux compris à la lecture des exemples qui suivent. Ces exemples sont donnés à titre indicatif, sans caractère limitatif.

La figure 1 représente les quantités de pinocembrine et galangine présentes dans l'extrait de *Propolis* mesurées dans des urines humaines après un régime de 6 et 8 gélules comprenant une composition selon l'invention.

La figure 2 représente l'effet d'une composition selon l'invention sur l'expression du gène *papG3* d'une souche d'*E.coli* uropathogène.

La figure 3 représente une courbe de bactéricidie de la *Propolis* et du ceftriaxone.

La figure 4 représente une courbe de bactéricidie de la *Propolis* et du cefixime.

**Exemple 1 : Production d'un extrait de cranberry *Vaccinium macrocarpon* :**

Un extrait de cranberry *Vaccinium macrocarpon* comprenant au moins 20% de PAC est obtenu selon l'exemple 1 de la demande de brevet EP 1 913 951.

**Exemple 2 : Production d'un extrait de *Propolis* :**

Après avoir récolté la *Propolis* brute, celle-ci subit une épuration, afin d'éliminer la cire, le bois ou les morceaux d'insectes éventuels. Cette phase d'épuration s'opère notamment par un nettoyage par des solvants appropriés, tel que de l'alcool. Puis la *Propolis* ainsi épurée est mise en solution hydro-alcoolique, filtrée, évaporée puis séchée. Elle est ensuite broyée et mélangée avec de la poudre de caroube en tant que support. L'extrait de *Propolis* ainsi obtenu se présente sous la forme d'une poudre micronisée de couleur beige qui contient 18% de matière active.

Exemple 3 : gélules

| | |
|---|---|
| - Extrait de cranberry *Vaccinium macrocarpon*(1) | 0,150 g |
| - Extrait de *Propolis* (2) | 0,250 g |

(1) Exocyan CRAN 20S commercialisé par la société Tournay Biotechnologies
(2) Propolis PPM 18 commercialisé par la société Lustrel

La composition selon l'exemple 3 se présente sous forme de poudre et est conditionnée en gélules.

**Exemple 4 : poudre en sachet-dose.**

| | |
|---|---|
| - Extrait de cranberry *Vaccinium macrocarpon*(1) | 0,3 g |
| - Extrait de *Propolis* (2) | 0,5 g |
| - Vitamine C | 0,060 g |

(1) Exocyan CRAN 20S commercialisé par la société Tournay Biotechnologies
(2) Propolis PPM 18 commercialisé par la société Lustrel

La poudre est conditionnée en sachets-dose et la posologie d'utilisation est de 2 à 4 sachets par jour pendant 2 à 7 jours.
La poudre peut également être conditionnée en gélules et la posologie d'utilisation est de 2 à 4 gélules par jour pendant 2 à 7 jours, à renouveler si besoin.

**Exemple 5 : gélules.**

| | |
|---|---|
| - Extrait de cranberry *Vaccinium macrocarpon*(1) | 0,150 g |
| - Extrait de *Propolis* (2) | 0,250 g |
| - Vitamine C | 0,030g |

(1) Exocyan CRAN 20S commercialisé par la société Tournay Biotechnologies
(2) Propolis PPM 18 commercialisé par la société Lustrel

La composition selon l'exemple 5 se présente sous forme de poudre et est conditionnée en gélules et la posologie d'utilisation est de 2 à 4 gélules par jour pendant la période de risque, à renouveler si besoin.

**Exemples 6 et 7 : compositions destinées au traitement et/ou à la prévention des infections vésicales et des carences en zinc chez l'homme**

| | **Exemple 6** | **Exemple 7** |
|---|---|---|
| Extrait de cranberry *Vaccinium macrocarpon*(1) | 0,3 g | 0,150 g |
| Extrait de *Propolis*(2) | 0,5 g | 0,250 g |
| Glycérine | qsp | qsp |
| Fructose | qsp | qsp |
| Lactose | qsp | qsp |
| Sulfate de Zinc | 0,0055 g | 0,0055 g |
| Arôme de fruits rouges | qsp | qsp |
| (1) Exocyan CRAN 20S commercialisé par la société Tournay Biotechnologies<br>(2) Propolis PPM 18 commercialisé par la société Lustrel | | |

La composition selon l'exemple 6 se présente sous forme de poudre et est conditionnée en sachets-dose ou en gélules.
La posologie d'utilisation en sachets-dose est de 2 à 4 sachets par jour pendant 2 à 7 jours.
La posologie d'utilisation en gélules est de 2 à 4 gélules par jour, à renouveler si besoin.
La composition selon l'exemple 7 se présente sous forme de poudre et est conditionnée en gélules.
La posologie d'utilisation en traitement flash est de 4 à 8 gélules pendant une période maximale d'une semaine.
La posologie d'utilisation en traitement préventif est de 2 à 4 gélules pendant une période de 15 jours, à renouveler si besoin.

**Exemples 8 et 9 : compositions destinées au traitement et/ou à la prévention des infections urinaires et des carences en fer chez la femme**

| | **Exemple 8** | **Exemple 9** |
|---|---|---|
| Extrait de cranberry *Vaccinium macrocarpon*(1) | 0,3 g | 0,150 g |
| Extrait de *Propolis*(2) | 0,5 g | 0,250 g |
| Glycérine | qsp | qsp |
| Fructose | qsp | qsp |
| Lactose | qsp | qsp |
| Sulfate de Fer | 0,00914g | 0,00914 g |
| Arôme de fruits rouges | qsp | qsp |
| (1) Exocyan CRAN 20S commercialisé par la société Tournay Biotechnologies<br>(2) Propolis PPM 18 commercialisé par la société Lustrel | | |

La composition selon l'exemple 8 se présente sous forme de poudre et est conditionnée en sachets-dose ou en gélules.
La posologie d'utilisation en sachets-dose est de 2 à 4 sachets par jour pendant 2 à 7 jours.
La posologie d'utilisation en gélules est de 2 à 4 gélules par jour, à renouveler si besoin.
La composition selon l'exemple 9 se présente sous forme de poudre et est conditionnée en gélules.

La posologie d'utilisation en traitement flash est de 4 à 8 gélules pendant une période maximale d'une semaine.
La posologie d'utilisation en traitement préventif est de 2 à 4 gélules pendant une période de 15 jours, à renouveler si besoin.

**Exemples 10 et 11 : gélules**

| | **Exemple 10** | **Exemple 11** |
|---|---|---|
| Extrait de cranberry *Vaccinium macrocarpon*(1) | 0,150 g | 0,150 g |
| Extrait de *Propolis*(2) | 0,100 g | 0,100 g |
| Cellulose (enveloppe) | qsp | qsp |
| Carbonate de calcium | qsp | qsp |
| Stearate de magnesium | qsp | qsp |
| Ethylvanilline | qsp | qsp |
| Sulfate de Zinc | 0,00125 g | |
| Sulfate de Fer | | 0,00125 g |
| Arômes menthol | qsp | qsp |
| (1) Exocyan CRAN 20S commercialisé par la société Tournay Biotechnologies<br>(2) Extrait de *Propolis* à 60% de *Propolis* pure commercialisé par la société Claudine Vallée | | |

La posologie d'utilisation de la composition selon l'exemple 10 est de 4 gélules par jour pendant 5 à 10 jours en utilisation d'attaque et de 2 gélules par jour pendant 10 jours à renouveler régulièrement en utilisation d'entretien.
La posologie d'utilisation de la composition selon l'exemple 11 est de 4 gélules par jour pendant 5 à 10 jours en utilisation d'attaque et de 2 gélules par jour pendant 10 jours à renouveler régulièrement en utilisation d'entretien.

**Exemple 12 : quantification des polyphénols de *Propolis* dans des urines humaines après ingestion d'une composition selon l'invention**

Deux volontaires sains âgés de plus de 18 ans, avec contraception efficace et ayant suivi une alimentation normale, ont suivi un régime comprenant l'ingestion de gélules selon la composition de l'exemple 3.
Deux régimes ont été suivis :

- prise de 6 gélules,
- prise de 8 gélules.

La prise de gélules s'est effectuée le matin à 8h et les urines ont été collectées par miction dans un flacon stérile avant ingestion, 1-6h après l'ingestion, 12h après l'ingestion et 24h après l'ingestion.
Les urines collectées sont hydrolysées par hydrolyse enzymatique (glucuronidase et sulfatase), puis les polyphénols sont extraits et purifiés sur Sep-Pack.
Les polyphénols de la *Propolis* comme la pinocembrine et la galangine sont ensuite caractérisés et dosés.
Les résultats sont présentés dans le tableau 1 ci-dessous ainsi que dans la figure 1.

Tableau 1

| | 1-6h (6 gélules) | 12h (6 gélules) | 24h (6 gélules) | 24h (8 gélules) |
|---|---|---|---|---|
| Pinocembrine (P) en mg/l | 3,344 | 1,141 | 0,407 | 1,368 |
| Galangine (G) en mg/l | 0,555 | 0,291 | 0,157 | 0,311 |

Ces résultats montrent que l'action de la *Propolis* présente dans la composition selon l'invention a effectivement lieu dans le système urinaire humain et démontre ainsi la pertinence de l'utilisation de la composition selon l'invention pour cibler le traitement de pathologies urinaires.

**Exemple 13 : étude de l'effet anti-adhérentiel d'une composition selon l'invention**

Quatre volontaires sains âgés de plus de 18 ans, avec contraception efficace, ayant suivi une alimentation normale et n'ayant pas perçu de traitement antibiotique dans les deux semaines précédentes et au long de l'étude ont suivi un régime comprenant l'ingestion de gélules selon la composition de l'exemple 3.

Deux régimes ont été suivis :

- prise de 4 gélules pour 2 volontaires,
- prise de 6 gélules pour les 2 autres volontaires.

La prise de gélules s'est effectuée le matin à 8h et les urines ont été collectées par miction dans un flacon stérile avant ingestion, 1-6h après l'ingestion, 12h après l'ingestion pour les 2 volontaires ayant ingéré 6 gélules et 24h après l'ingestion.

Les 2 volontaires ayant pris 4 gélules ont donc eu 3 recueils urinaires (H0, H1-6 et H24), soit 6 urines à analyser pour ce régime. S'il y avait différentes mictions (entre 1 et 6h après la prise), elles ont été mélangées dans un même flacon.

Les 2 volontaires ayant pris 6 gélules ont donc eu 4 recueils urinaires (H0, H1-6, H12 et H24), soit 8 urines à analyser pour ce régime. S'il y avait différentes mictions (entre 1 et 6h après la prise), elles ont été mélangées dans un même flacon.

Différents paramètres biologiques et physico-chimiques des échantillons d'urines ont été déterminés à l'aide du système Multistix®. Une abondance de leucocytes et/ou d'hématies et/ou une positivité aux nitrites auraient entraînées l'exclusion de l'échantillon d'urines. Ces échantillons ont alors été réfrigérés à +4°C, centrifugés à 4000g pendant 15 minutes à +4°C, puis immédiatement stockés à 20°C.

Une souche d'*E. coli* uropathogène précédemment isolée des urines d'un patient ayant une infection urinaire a été sélectionnée: NECS19923 possédant des fimbriae de type P (*papG*) et des pili de type-1. Cette souche a été préalablement modifiée génétiquement par insertion d'un plasmide portant le gène codant pour la GFP (green fluorescence protein).

Des expériences d'adhésion ont été réalisées sur des lignées de cellules urothéliales de type T24 d'origine humaine (ATCC HTB-4), adaptées de Di Martino et al. Les bactéries ont été mises en présence des urines des volontaires saines dans un milieu de culture contenant 5% de *Luria Bertani* (5% (v/v)) durant une nuit à 37°C sous agitation. Les bactéries ont été ensuite centrifugées et resuspendues à une concentration de 108 CFU/ ml dans un milieu de McCoy, puis mises en contact avec une monocouche de cellules urothéliales T24 et incubées 3h à 37 °C. Après 6 lavages avec du PBS (Phosphate Buffered Saline), les cellules ont été fixées avec du méthanol. Les lames ont ensuite été examinées sous microscope à fluorescence, les bactéries ont été alors visualisées en vert. Le nombre moyen de bactéries adhérentes par cellule a été déterminé en examinant 100 cellules urothéliales et représente un index adhésion. Cet index est exprimé comme la médiane de 4 tests indépendants.

Les résultats sont présentés dans le tableau 2 ci-dessous

Tableau 2

| | Médiane AI [range] | | | |
|---|---|---|---|---|
| | Volontaire 1 (4 gélules) | Volontaire 2 (4 gélules) | Volontaire 3 (6 gélules) | Volontaire 4 (6 gélules) |
| Oh | 20,4 [16-26] | 18,6 [15-28] | 23,0 [14-28] | 21,2 [15-25] |
| 1-6h | 3,7 [2-10] | 2,1 [1-9] | 3,0 [0-5] | 3,1 [0-4] |
| 12h | | | 2,1 [0-5] | 2,2 [0-5] |
| 24h | 17,7 [14-22] | 15,8 [11-18] | 16,9 [12-21] | 19,1 [10-25] |

Ces résultats montrent que la composition selon l'invention présente une activité anti-adhérentielle à l'encontre de *E.Coli* "immédiate" mais également que cette activité se maintient dans le temps, jusqu'à 24h.

**Exemple 14 : étude *ex vivo* de l'influence d'une composition selon l'invention sur la virulence d'une souche d'*E.coli* uropathogène**

Deux volontaires sains âgés de plus de 18 ans, avec contraception efficace, ayant suivi une alimentation normale et n'ayant pas perçu de traitement antibiotique dans les deux semaines précédentes et au long de l'étude ont suivi un régime comprenant l'ingestion de gélules selon la composition de l'exemple 3.

Trois régimes ont été suivis :

- prise de 4 gélules,
- prise de 6 gélules,
- prise de 8 gélules.

La prise de gélules s'est effectuée le matin à 8h et les urines ont été collectées par miction dans un flacon stérile avant ingestion, 1-6h après l'ingestion, 12h après l'ingestion et 24h après l'ingestion.

Les 2 volontaires ont donc eu 4 recueils urinaires (H0, H1-6, H12 et H24), soit 8 urines à analyser pour ce régime. S'il y avait différentes mictions (entre 1 et 6h après la prise), elles ont été mélangées dans un même flacon.

Différents paramètres biologiques et physico-chimiques des échantillons d'urines ont été déterminés à l'aide du système Multistix®. Une abondance de leucocytes et/ou d'hématies et/ou une positivité aux nitrites auraient entraînées l'exclusion de l'échantillon d'urines. Ces échantillons ont alors été réfrigérés à +4°C, centrifugés à 4000g pendant 15 minutes à +4°C, puis immédiatement stockés à 20 °C. Une période de "wash-out" d'une semaine entre les changements de régime a été respectée.

Une souche d'*E. coli* uropathogène précédemment isolée des urines d'un patient ayant une infection urinaire a été sélectionnée: NECS19923 possédant des fimbriae de type P (*papG*) et des pili de type-1. Cette souche a été préalablement modifiée génétiquement par insertion d'un plasmide portant le gène codant pour la GFP (green fluorescence protein).

L'étude *in vivo* de la cytotoxicité d'*E. coli* a été réalisée sur le modèle *Caenorhabditis elegans* selon la méthode décrite par Kurz et al. à l'exception des vers utilisés qui est une lignée de mutant Fer-15, qui ont une fertilité dépendante de la température ambiante. Les mutants Fer-15 sont fournis par le *Caenorhabditis* Genetics Center (USA). Pour synchroniser la croissance des nématodes, les oeufs ont été collectés en utilisant la méthode à l'hypochlorite. Des géloses NGM (Nematode Growth Medium) ont été inoculées avec 10 μl d'une culture de 18h d'*E. coli* mises en présence de la composition selon l'exemple 3. Ces géloses ont été ensuite incubées à 37 °C durant 8-10 h, puis ensemencées avec des nématodes au stade larvaire L4 (20 à 30 par gélose). Les géloses ont été ensuite incubées à 25 °C et chaque jour le nombre de vers vivants a été dénombré sous stéréomicroscope (Leica MS5). Ces expériences ont permis d'établir des courbes de survie (DL50: Temps de demi-vie et Durée de vie) et ont démontré la présence, l'absence ou la diminution de la virulence (cytotoxicité) des souches d'*E. coli.* Pour chaque expérience, les souches ont été testées sur 3 expériences indépendantes et ces expériences ont été répétées 4 fois pour chaque test. Un nématode est considéré comme mort quand il ne répond plus au toucher par une öse métallique. Les vers venant se coller contre la paroi des boîtes de Pétri et qui décèdent ont été exclus de l'analyse. Pour comparer les durées de vie des nématodes entre les différents régimes, le modèle de régression de Cox a été appliqué. Afin d'effectuer des comparaisons par paire, un log rank test a été utilisé. Les analyses ont été effectuées grâce au logiciel SAS®/ETS release 9.1 (SAS Institute Inc, Cary, NC, USA).

Les résultats sont présentés dans le tableau 3 ci-dessous

Tableau 3

| Tests | LT50 (jours) | LT100 (jours) |
|---|---|---|
| Urines T0 | 4.2 ±0.2 | 7.4 ±0.6 |
| Urines 4 gélules T1-6h | 6.0 ±0.1 | 11.3 ±0.7 |
| Urines 4 gélules T12h | 5.5 ±0.2 | 9.3 ±0.7 |
| Urines 4 gélules T24h | 5.0 ±0.2 | 7.8 ±0.8 |
| Urines 6 gélules T1-6h | 6.3 ±0.3 | 11.3 ±0.7 |
| Urines 6 gélules T12h | 5.3 ±0.3 | 9.5 ±0.5 |
| Urines 6 gélules T24h | 5.8 ±0.2 | 9.0 ±1.0 |
| Urines 8 gélules T1-6h | 6.0 ±0.1 | 11.8 ±0.8 |
| Urines 8 gélules T24h | 6.3 ±0.3 | 11.5 ±0.5 |
| Souche contrôle OP50 | 7.5 ±0.5 | 13.3 ±0.7 |

Les résultats montrent que la composition selon l'invention permet de réduire la virulence d'une souche d'*E.coli* et également que cette activité se maintient dans le temps.

**Exemple 15 : évaluation de l'expression du gène *papG3* sous l'effet d'une composition selon l'invention**

Une souche d'*Escherichia coli* uropathogène isolée au cours d'une infection urinaire a été sélectionnée au sein du laboratoire de l'équipe INSERM Espri 6 : il s'agit d'une souche sensible aux antibiotiques : NECC853118, *papG3+,* phylotype B2.

Les urines de volontaires sains âgés de plus de 18 ans, avec contraception efficace, ayant suivi une alimentation normale et n'ayant pas perçu de traitement antibiotique dans les deux semaines précédentes et au long de l'étude ont été recueillies après les régimes suivants :

- placebo
- 4 gélules, 1-6h après ingestion
- 4 gélules, 12h après ingestion,
- 8 gélules, 1-6h après ingestion,
- 8 gélules, 24h après ingestion,

chaque gélule correspondant à la composition selon l'exemple 3.

La mise en culture de la souche d'*E. coli* dans les différentes urines recueillies après les différents régimes s'effectue durant 18h à 37°C sous agitation.

Le niveau d'expression du gène *papG3 a* été déterminé par quantitative real-time RT-PCR (Real Time - Polymerase Chain Reaction). L'expression des transcrits a été normalisée avec le gène de ménage codant pour la GAPDH (Glyceraldehyde-3 Phosphate Deshydrogenase) mesuré sur chaque échantillon. Pour prévenir la dégradation des ARNm extraits après la lyse cellulaire (ce qui peut altérer l'expression des gènes), les ARN totaux ont été isolés dans un environnement RNAse-free en utilisant le kit RNeasy Protect Mini (Qiagen, Hilden, Germany) en suivant les recommandations du fournisseur. L'intégrité, la pureté et la concentration des ARNm extraits ont été mesurées par spectrophotométrie à 260 nm. L'ARN purifié a été conservé dans de l'eau stérile sans RNase dans des tubes Eppendorf de congélation. La quantification des échantillons a été effectuée sur un LC480 (Roche) en utilisant le kit QuantiTect SYBR Green RT-PCR Kit (Qiagen, Hilden, Germany). Le gène de ménage *gapdh* est utilisé comme une référence de l'expérience et parce qu'il a un niveau d'expression constant sous les conditions utilisées. L'efficacité de l'amplification du gène cible et du gène référence a été déterminée à partir de l'amplification d'une série de dilution des échantillons et les conditions PCR ont été optimisées jusqu'à l'obtention d'une efficacité comparable entre les séries (efficacité = 2). Les amplifications du *gapdh* et de *papG3* ont été réalisées dans des tubes séparés en utilisant la même quantité d'ARN totaux. Les quantités d'ARNm de chaque gène ont été déterminées par la comparaison des seuils d'amplification (CT).

Les résultats ont été exprimés par une sous/sur-expression de *papG3* par rapport à l'expression de ce gène dans des conditions normales (*E. coli* dans urines sans PAC) et sont présentés dans la figure 2.

Par rapport à l'expression du gène *papG3* détectée dans la souche d'*E. coli* mis en contact avec des urines témoins, ce même gène est :

- près de 70% (69%) moins exprimé dans la souche d'*E. coli* mise en contact avec des urines à 8 gélules de composition selon l'invention prélevées à 1-6h,
- près de 50% (47%) moins exprimé dans la souche d'*E. coli* mise en contact avec des urines à 8 gélules de composition selon l'invention prélevées à 24h,
- 35% moins exprimé dans la souche d'*E. coli* mise en contact avec des urines à 4 gélules de composition selon l'invention prélevées à 1-6h,
- près de 20% moins exprimé (18%) dans la souche d'*E. coli* mise en contact avec des urines à 4 gélules de composition selon l'invention prélevées à 24h.

Ces résultats montrent que la composition selon l'invention permet de diminuer l'expression du gène *papG3* et que cette activité se maintient dans le temps. Ils confirment ainsi l'effet de la composition selon l'invention sur la diminution de la virulence d'*E.coli.*

**Exemple 16** (illustratif) : **étude *in vitro* de l'effet anti-bactérien d'une composition et de la synergie avec différents agents antibiotiques utilisés en pathologie urinaire**

Six souches d'*Escherichia coli* d'origine différentes (cystites, pyélonéphrites aiguës, portage chronique urinaire), de sensibilités différentes aux principaux antibiotiques habituellement utilisés dans les infections urinaires et de virulences différentes ont été sélectionnées au sein du souchier du laboratoire de l'équipe INSERM Espri 26 :

- 2 souches sensibles aux ATB : NECS892841 et NECS30090

- 2 souches résistantes aux quinolones : NECS858785 et NECS864598
- 2 souches sécrétrices de BLSE : NEC892420 et NEC118564

Trois compositions ont été évaluées :

- une composition comprenant de la poudre de *Propolis* sur dextrine maltose
- une composition comprenant de la poudre de *Propolis* sur dextrine maltose et des PAC de type A
- une composition comprenant de la dextrine maltose

Trois antibiotiques ont été étudiés :

- ofloxacine
- ceftriaxone
- cefixime

L'étude *in vitro* de l'effet antibactérien se divise en deux étapes :

- une étape de détermination de la Concentration Minimale Inhibitrice (CMI) de chaque composition (gamme de concentrations étudiées : 0,5 à 5196 mg/L) et de l'antibiotique par méthode de dilution en milieu liquide selon les recommandations du CA-SFM (Comité de l'Antibiogramme de la Société Française de Microbiologie).
- une étape de détermination de la CMI de l'antibiotique en présence de chaque composition en concentration sub-inhibitrice (120 ou 250 mg/L en fonction des souches) (gamme de concentrations étudiées : 0,5 à 512 mg/L) par méthode de dilution en milieu liquide selon les recommandations du CA-SFM.

L'ensemble des tubes a été mis à l'étuve à 37°C sans agitation durant 1 nuit.
La $CMI_{50}$ est la plus petite concentration d'antibiotique qui inhibe toute culture visible d'une souche bactérienne après 18 heures de culture à 37°C.
Pour l'ofloxacine, une CMI> 1mg/l équivaut à une souche résistante.
Pour la ceftriaxone, une CMI> 2mg/l équivaut à une souche résistante.
Pour la cefixime, une CMI> 2mg/l équivaut à une souche résistante.
La sensibilité ou la résistance à l'ofloxacine, à la ceftriaxone ou à la cefixime est interprétée en fonction des recommandations du CA-SFM.
Les résultats sont présentés dans les tableaux 4, 5 et 6 ci-dessous :

Tableau 4

| **Souches *E.coli*** | **Ofloxacine** | **Ofloxacine + Maltose dextrine** | **Ofloxacine + Propolis** | **Ofloxacine + Propolis + PAC type A** |
|---|---|---|---|---|
| S | 1 | 1 | 0.5 | 0.5 |
| S | 0.25 | 0.25 | 0.06 | 0.06 |
| OFX R | 2 | 2 | 0.5 | 0.5 |
| OFX R | 32 | 32 | 8 | 8 |
| BLSE | >32 | >32 | 8 | 8 |
| BLSE | >32 | > 32 | 16 | 16 |
| S : souche sensible aux antibiotiques communément testés contre *E. coli* ; OFX R : souche résistante à l'ofloxacine ; BLSE : souche productrice de b-lactamase à spectre étendu | | | | |

Tableau 5

| **Souches *E.coli*** | **Ceftriaxone** | **Ceftriaxone + Maltose dextrine** | **Ceftriaxone + Propolis** | **Ceftriaxone + Propolis + PAC type A** |
|---|---|---|---|---|
| S | 1 | 1 | 0.125 | 0.125 |
| S | 0.5 | 0.5 | 0.06 | 0.06 |

(suite)

| Souches *E.coli* | Ceftriaxone | Ceftriaxone + Maltose dextrine | Ceftriaxone + Propolis | Ceftriaxone + Propolis + PAC type A |
|---|---|---|---|---|
| OFX R | 0.25 | 0.25 | <0.06 | <0.06 |
| OFX R | 0.5 | 0.5 | 0.06 | 0.06 |
| BLSE | >32 | >32 | 2 | 2 |
| BLSE | 32 | 32 | 2 | 1 |
| S : souche sensible aux antibiotiques communément testés contre *E. coli* ; OFX R : souche résistante à l'ofloxacine ; BLSE : souche productrice de b-lactamase à spectre étendu | | | | |

Tableau 6

| Souches *E.coli* | Cefixime | Cefixime + Maltose dextrine | Cefixime + Propolis | Cefixime + Propolis + PAC type A |
|---|---|---|---|---|
| S | 1 | 1 | 0.25 | 0.25 |
| S | 0.5 | 0.5 | 0.25 | 0.125 |
| OFX R | 0.5 | 0.5 | 0.125 | 0.125 |
| OFX R | 1 | 1 | 0.125 | 0.25 |
| BLSE | 16 | 16 | 8 | 8 |
| BLSE | 8 | 8 | 2 | 2 |
| S : souche sensible aux antibiotiques communément testés contre *E. coli* ; OFX R : souche résistante à l'ofloxacine ; BLSE : souche productrice de b-lactamase à spectre étendu | | | | |

Les résultats montrent que la *Propolis* permet d'améliorer l'activité de chacun des antibiotiques évalués et démontre ainsi un effet synergique clair de la *Propolis* avec les 2 classes d'antibiotiques (céphalosporine, fluoroquinolone) auxquelles appartiennent les antibiotiques évalués.

Par ailleurs, ces résultats montrent également que les PAC de type A ne nuisent pas à la synergie d'activité entre la *Propolis* et l'antibiotique.

**Exemple 17 (illustratif) : étude complémentaire de la synergie entre *Propolis* et différents agents antibiotiques utilisés en pathologie urinaire - courbes de bactéricidie**

Une souche d'*Escherichia coli* isolée au cours d'une infection urinaire a été sélectionnée au sein du souchier du laboratoire de l'équipe INSERM Espri 26 : souche sensible aux antibiotiques NECS892841.

Deux compositions ont été évaluées :

- une composition comprenant de la poudre de *Propolis* sur dextrine maltose
- une composition comprenant de la dextrine maltose (témoin)

2 antibiotiques sont évalués :

- ceftriaxone (CRO)
- cefixime (CFM)

L'établissement de l'effet antibactérien de chaque composition associée au ceftriaxone se base sur les expériences suivantes.

Les expériences ont été réalisées dans 20 ml de Mueller-Hinton sous agitation à 37 °C. Chaque composition a été évaluée à 1x la CMI (256 mg/L), le ceftriaxone à 0.5x la CMI (0.5 mg/L) et les échantillons ont été étalés pour un comptage des colonies (CFU) à 0, 1, 2, 3, 5, 6 et 24h. Une réduction ≥ 3-log par rapport à l'inoculum d'origine à 24h sera considérée comme bactericide.

L'établissement de l'effet antibactérien de chaque composition associée au céfixime se base sur les expériences suivantes.

Les expériences ont été réalisées dans 20 ml de Mueller-Hinton sous agitation à 37 °C. Chaque composition a été évaluée à 1x la CMI (256 mg/L), le céfixime à 0.5x la CMI (0.5 mg/L) et les échantillons ont été étalés pour un comptage des colonies (CFU) à 0, 1, 2, 3, 5, 6 et 24h. Une réduction ≥ 3-log par rapport à l'inoculum d'origine à 24h sera considérée comme bactericide.

Le dénombrement des colonies bactériennes (CFU) a été réalisé après 18 heures de culture à 37 °C.

Les courbes de bactéricidie sont présentées aux figures 3 et 4.

Les courbes de bactéricidie des figures 3 et 4 permettent de confirmer la synergie d'activité entre la *Propolis* et chacun des antibiotiques évalués.

**Revendications**

1. Composition alimentaire antibactérienne comprenant un extrait de cranberry *Vaccinium macrocarpon* et un extrait de *Propolis,* dans laquelle :

    - l'extrait de *Propolis* comprend de l'acide caféique, de l'acide férulique, de la galangine et de la pinocembrine,
    - l'extrait de cranberry *Vaccinium macrocarpon* contient une proportion en poids de proanthocyanidines (PAC) supérieure ou égale à 10%, de préférence supérieure à 10%, avantageusement comprise entre 20 et 50%, ladite proportion étant mesurée par la méthode de dosage par la Vanilline

    la composition étant **caractérisée par** un ratio en poids extrait de *Propolis* : extrait de cranberry *Vaccinium macrocarpon* compris entre 1 :2 et 2 :1

2. Composition selon la revendication précédente **caractérisée en ce que** la teneur en poids d'extrait de *Propolis* est comprise entre 10 et 80%, de préférence entre 30 et 70%.

3. Composition selon l'une quelconque des revendications précédentes **caractérisée en ce que** l'extrait de cranberry *Vaccinium macrocarpon* est obtenu par un procédé de concentration et d'isolement des fractions polyphénoliques du jus de cranberry *Vaccinium macrocarpon.*

4. Composition selon l'une quelconque des revendications précédentes **caractérisée en ce que** la teneur en poids d'extrait de cranberry *Vaccinium macrocarpon* est comprise entre 10 et 80%, de préférence entre 25 et 70%.

5. Composition selon l'une quelconque des revendications précédentes **caractérisée en ce qu'**elle comprend également un composé choisi dans le groupe constitué par les actifs pharmaceutiques, les ingrédients alimentaires et les nutriments.

6. Composition selon la revendication précédente **caractérisée en ce que** l'ingrédient alimentaire est choisi dans le groupe constitué par le zinc et ses dérivés, de préférence parmi les sels de zinc.

7. Composition selon la revendication 5 **caractérisée en ce que** l'ingrédient alimentaire est choisi dans le groupe constitué par le fer et ses dérivés, de préférence parmi les sels de fer.

8. Composition selon les revendications 6 ou 7 **caractérisée en ce que** :

    - la teneur en poids de zinc ou d'un de ses dérivés est comprise entre 0,01 et 2%, de préférence entre 0,1 et 1,5%, ou
    - la teneur en poids de fer ou d'un de ses dérivés est comprise entre 0,05 et 3%, de préférence entre 0,5 et 2,5%.

9. Composition selon la revendication 5 **caractérisée en ce que** l'ingrédient alimentaire est choisi dans le groupe constitué par la vitamine C et ses dérivés, de préférence parmi les sels d'acide ascorbique, avantageusement l'ascorbate de sodium.

10. Composition selon la revendication 9 **caractérisée en ce que** la teneur en poids de vitamine C ou d'un de ses dérivés est comprise entre 1 et 10%, de préférence entre 2 et 8%.

**11.** Complément alimentaire comprenant une composition selon l'une quelconque des revendications 1 à 10 pour l'utilisation dans le traitement et/ou la prévention des infections bactériennes aigues ou chroniques ou des bactériuries asymptomatiques.

**12.** Médicament comprenant une composition selon l'une quelconque des revendications 1 à 10 pour l'utilisation dans le traitement et/ou la prévention des infections bactériennes aigues ou chroniques ou des bactériuries asymptomatiques.

**13.** Composition selon la revendication 5 **caractérisée en ce que** l'actif pharmaceutique est un antibiotique.

**14.** Kit comprenant

- une première composition selon l'une quelconque des revendications 1 à 10,
- une seconde composition comprenant un principe actif pharmaceutique notamment antibiotique ou antioxydant comme la vitamine C et ses dérivés ou un ingrédient alimentaire comme le zinc, le fer et leurs dérivés ou un nutriment.

**15.** Composition comprenant une composition selon l'une quelconque des revendications 1 à 10 et un antibiotique, pour une administration simultanée, séparée ou étalée dans le temps, pour l'utilisation dans le traitement et/ou la prévention des infections bactériennes aigues ou chroniques ou des bactériuries asymptomatiques.

**16.** Kit ou composition selon l'une des revendications 14 et 15, comprenant un antibiotique qui est une céphalosporine ou une fluoroquinolone, de préférence choisie dans le groupe consistant en ceftriaxone, cefixime et ofloxacine.

**Patentansprüche**

**1.** Antibakterielle Nahrungsmittelzusammensetzung umfassend einen Auszug der Moosbeere *Vaccinium macrocarpon* und einen Auszug von *Propolis,* wobei:

- der Auszug von *Propolis* Kaffeesäure, Ferulasäure, Galangin und Pinocembrin umfasst,
- der Auszug der Moosbeere *Vaccinium macrocarpon* einen Gewichtsanteil an Proanthocyanidinen (PAC) von mehr als oder gleich 10 %, vorzugsweise mehr als 10 %, vorteilhaft zwischen 20 und 50 % enthält, wobei der Anteil mittels der Dosierungsmethode durch Vanillin gemessen wurde,

wobei die Zusammensetzung durch ein Gewichtsverhältnis von Auszug von *Propolis* : Auszug der Moosbeere *Vaccinium macrocarpon* zwischen 1:2 und 2:1 gekennzeichnet ist.

**2.** Zusammensetzung gemäß dem voranstehenden Anspruch, **dadurch gekennzeichnet, dass** der Gewichtsgehalt des Auszugs von *Propolis* zwischen 10 und 80 %, vorzugsweise zwischen 30 und 70 % beträgt.

**3.** Zusammensetzung gemäß einem der voranstehenden Ansprüche, **dadurch gekennzeichnet, dass** der Auszug der Moosbeere *Vaccinium macrocarpon* mittels eines Konzentrations- und Isolationsverfahren von polyphenolischen Fraktionen des Saftes der Moosbeere *Vaccinium macrocarpon* erhalten wurde.

**4.** Zusammensetzung gemäß einem der voranstehenden Ansprüche, **dadurch gekennzeichnet, dass** der Gewichtsgehalt des Auszugs der Moosbeere *Vaccinium macrocarpon* zwischen 10 und 80 %, vorzugsweise zwischen 25 und 70 % beträgt.

**5.** Zusammensetzung gemäß einem der voranstehenden Ansprüche, **dadurch gekennzeichnet, dass** sie ebenso eine Verbindung ausgewählt aus der Gruppe bestehend aus pharmazeutischen Wirkstoffen, Lebensmittelinhaltsstoffen und Nährstoffen umfasst.

**6.** Zusammensetzung gemäß einem der voranstehenden Ansprüche, **dadurch gekennzeichnet, dass** der Lebensmittelinhaltsstoff ausgewählt ist aus der Gruppe bestehend aus Zink und seinen Derivaten, vorzugsweise aus Zinksalzen.

**7.** Zusammensetzung gemäß Anspruch 5, **dadurch gekennzeichnet, dass** der Lebensmittelinhaltsstoff ausgewählt

ist aus der Gruppe bestehend aus Eisen und seinen Derivaten, vorzugsweise aus Eisensalzen.

**8.** Zusammensetzung gemäß den Ansprüchen 6 oder 7, **dadurch gekennzeichnet, dass**:

- der Gewichtsgehalt von Zink oder eines seiner Derivate zwischen 0,01 und 2 %, vorzugsweise zwischen 0,1 und 1,5 % beträgt, oder
- der Gewichtsgehalt von Eisen oder einem seiner Derivate zwischen 0,05 und 3 %, vorzugsweise zwischen 0,5 und 2,5 % beträgt.

**9.** Zusammensetzung gemäß Anspruch 5, **dadurch gekennzeichnet, dass** der Lebensmittelinhaltsstoff ausgewählt ist aus der Gruppe bestehend aus Vitamin C und seinen Derivaten, vorzugsweise aus den Salzen von Ascorbinsäure, vorteilhaft Natriumascorbat.

**10.** Zusammensetzung gemäß Anspruch 9, **dadurch gekennzeichnet, dass** der Gewichtsgehalt von Vitamin C oder eines seiner Derivate zwischen 1 und 10 %, vorzugsweise zwischen 2 und 8 % beträgt.

**11.** Nahrungsergänzungsmittel umfassend eine Zusammensetzung gemäß einem der Ansprüche 1 bis 10 zur Verwendung in der Behandlung und/oder Prävention von akuten oder chronischen bakteriellen Infektionen oder asymptotischen Bakteriurien.

**12.** Medikament umfassend eine Zusammensetzung gemäß einem der Ansprüche 1 bis 10 zur Verwendung in der Behandlung und/oder Prävention von akuten oder chronischen bakteriellen Infektionen oder asymptotischen Bakteriurien.

**13.** Zusammensetzung gemäß Anspruch 5, **dadurch gekennzeichnet, dass** der pharmazeutische Wirkstoff ein Antibiotikum ist.

**14.** Kit umfassend

- eine erste Zusammensetzung gemäß einem der Ansprüche 1 bis 10,
- eine zweite Zusammensetzung umfassend als pharmazeutischen Wirkstoff insbesondere ein Antibiotikum oder Antioxidans wie Vitamin C und seine Derivate oder einen Nahrungsmittelinhaltsstoff wie Zink, Eisen und ihre Derivate oder einen Nährstoff.

**15.** Zusammensetzung umfassend eine Zusammensetzung gemäß einem der Ansprüche 1 bis 10 und ein Antibiotikum, für eine simultane, getrennte oder zeitlich gestaffelte Verabreichung, zur Verwendung in der Behandlung und/oder Prävention von akuten oder chronischen bakteriellen Infektionen oder asymptotischen Bakteriurien.

**16.** Kit oder Zusammensetzung gemäß einem der Ansprüche 14 und 15, umfassend ein Antibiotikum, das ein Cephalosporin oder ein Fluorochinolon ist, vorzugsweise ausgewählt aus der Gruppe bestehend aus Ceftriaxon, Cefixim und Ofloxacin.

## Claims

**1.** An antibacterial food composition comprising a *Vaccinium macrocarpon* cranberry extract and a *Propolis* extract, wherein:

- the ***Propolis*** extract comprises caffeic acid, ferulic acid, galangin and pinocembrin,
- the *Vaccinium macrocarpon* cranberry extract contains a weight proportion of proanthocyanidins (PACs) greater than or equal to 10%, preferably greater than 10%, advantageously comprised between 20 and 50%, said proportion being measured by the vanillin assay method

the composition being **characterized by** a ***Propolis*** extract:*Vaccinium macrocarpon* cranberry extract weight ratio comprised between 1:2 and 2:1

**2.** The composition according to the preceding claim, **characterized in that** the *Propolis* extract weight content is comprised between 10 and 80%, preferably between 30 and 70%.

3. The composition according to any of the preceding claims, **characterized in that** the *Vaccinium macrocarpon* cranberry extract is obtained by a method for concentrating and isolating polyphenolic fractions from *Vaccinium macrocarpon* cranberry juice.

4. The composition according to any of the preceding claims, **characterized in that** the *Vaccuium macrocarpon* cranberry extract weight content is comprised between 10 and 80%, preferably between 25 and 70%.

5. The composition according to any of the preceding claims, **characterized in that** it also comprises a compound selected from the group formed by pharmaceutical actives, food ingredients and nutrients.

6. The composition according to the preceding claim, **characterized in that** the food ingredient is selected from the group formed by zinc and its derivatives, preferably from zinc salts

7. The composition according to claim 5, **characterized in that** the food ingredient is selected from the group formed by iron and its derivative, preferably from iron salts.

8. The composition according to claims 6 or 7, **characterized in that** :

- the weight content of zinc or of one of its derivatives is comprised between 0.01 and 2%, preferably between 0.1 and 1.5%, or
- the weight content of iron or of one of its derivatives is comprised between 0.05 and 3%, preferably between 0.5 and 2.5%

9. The composition according to claim 5, **characterized in that** the food ingredient is selected from the group formed by vitamin C and its derivatives preferably from ascorbic acid salts, advantageously the sodium ascorbate.

10. The composition according to claim 9, **characterized in that** the weight content of vitamin C or of one of its derivatives is comprised between 1 and 10%, preferably between 2 and 8%.

11. A food supplement comprising a composition according to any of claims 1 to 10 for use in treating and/or preventing acute or chronic bacterial infections or asymptomatic bacteriurias.

12. A drug comprising a composition according to any of claims 1 to 10 for use in treating and/or preventing acute or chronic bacterial infections of asymptomatic bacteriurias.

13. Composition according to claim 5, **characterized in that** the pharmaceutical active is an antibiotic.

14. A kit comprising:

- a first composition according to any one of claims 1 to 10,
- a second composition comprising a pharmaceutical active ingredient, notably an antibiotic or an antioxidant such as vitamin C and its derivatives or a food ingredient such as zinc, iron and their derivatives or a nutrient.

15. A composition comprising a composition according to any of claims 1 to 10 and an antibiotic for simultaneous, separate administration or spread out in time administration, for use in treating and/or preventing acute or chronic bacterial infections or asymptomatic bacteriurias.

16. The kit or composition according to one of claims 14 and 15, comprising an antibiotic which is a cephalosporin or a fluoroquinolone, preferably selected from the group consisting of ceftriaxone, cefixime and ofloxacin.

FIG 1

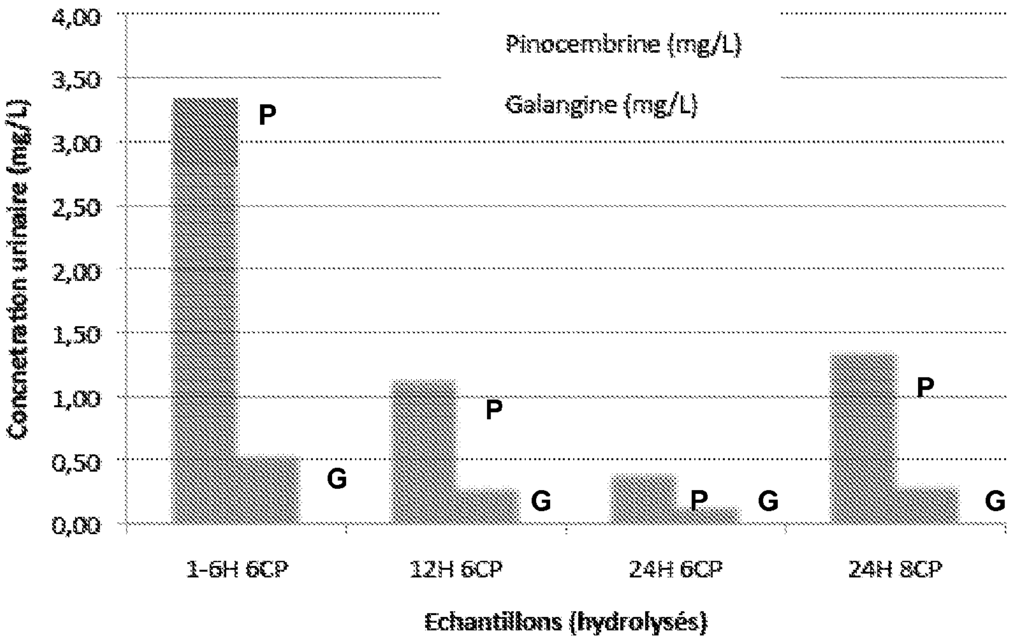
4,00
3,50
3,00
2,50
2,00
1,50
1,00
0,50
0,00
Concnetration urinaire (mg/L)
Pinocembrine (mg/L)
Galangine (mg/L)
P
G
P
G
P
G
P
G
1-6H 6CP
12H 6CP
24H 6CP
24H 8CP
Echantillons (hydrolysés)

FIG 2

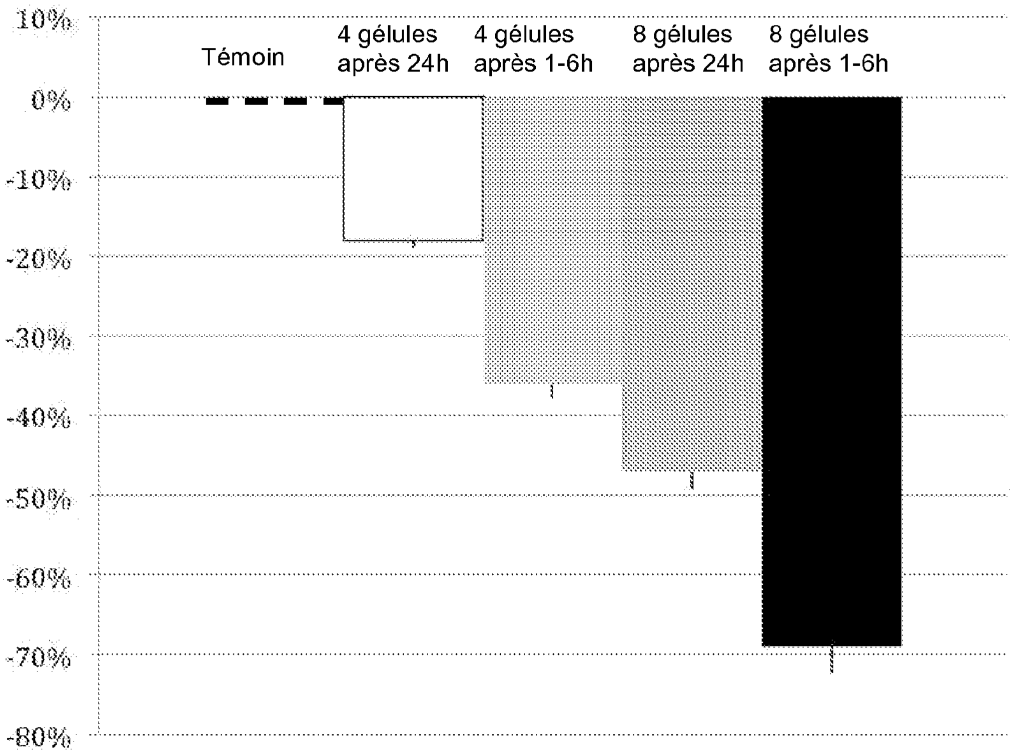
10%
0%
-10%
-20%
-30%
-40%
-50%
-60%
-70%
-80%
Témoin
4 gélules après 24h
4 gélules après 1-6h
8 gélules après 24h
8 gélules après 1-6h

FIG 3

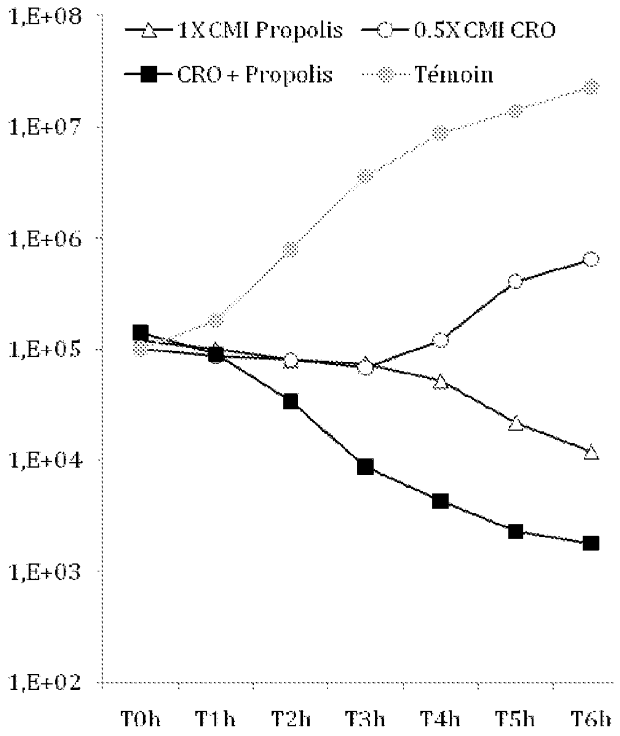
1,E+08
1,E+07
1,E+06
1,E+05
1,E+04
1,E+03
1,E+02
1X CMI Propolis
0.5X CMI CRO
CRO + Propolis
Témoin
T0h
T1h
T2h
T3h
T4h
T5h
T6h

FIG 4

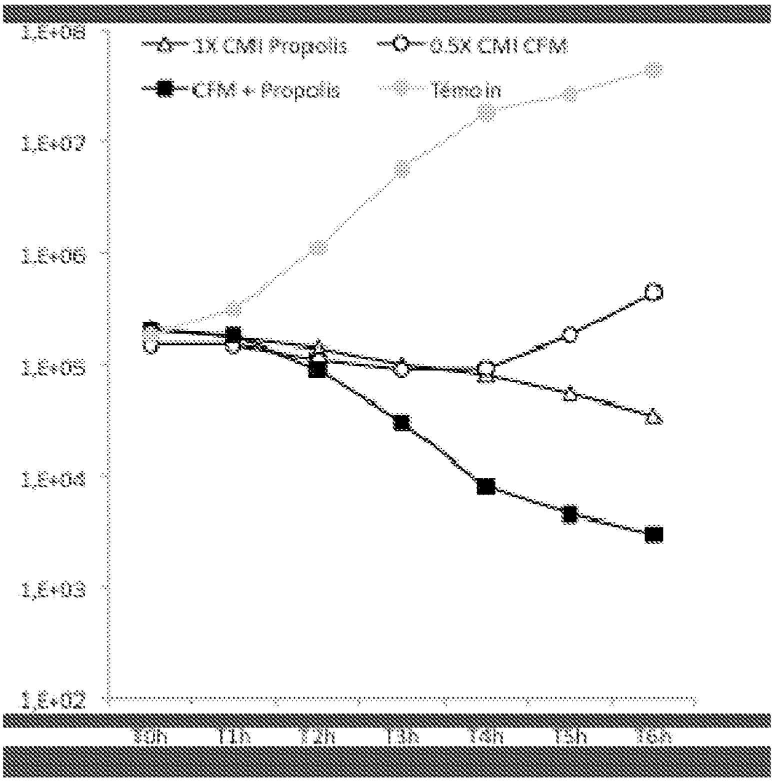
1,E+08
1,E+07
1,E+06
1,E+05
1,E+04
1,E+03
1,E+02
1X CMI Propolis
0.5X CMI CFM
CFM + Propolis
Témoin
T0h
T1h
T2h
T3h
T4h
T5h
T6h

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1902721 A
- EP 1913951 A


### Littérature non-brevet citée dans la description


- **JP LAVIGNE.** *Clin. Microbiol. Infect.,* 2007, 1-5